(19) **Europäisches Patentamt**

**European Patent Office**

**Office européen des brevets**

(11) Veröffentlichungsnummer: **0 004 037**
**B1**

(12) **EUROPÄISCHE PATENTSCHRIFT**

(45) Veröffentlichungstag der Patentschrift:
**13.04.83**

(21) Anmeldenummer: **79100611.7**

(22) Anmeldetag: **01.03.79**

(51) Int. Cl.³: **G 01 R 19/165,** A 61 N 1/08,
A 61 N 1/32, G 01 R 19/04

(54) Verfahren und Vorrichtung zur Messung von elektrischen Strömen oder Spannungen in einer Mehrzahl von Strom- bzw. Spannungskreisen.

(30) Priorität: **08.03.78 DE 2810046**

(43) Veröffentlichungstag der Anmeldung:
**19.09.79 Patentblatt 79/19**

(45) Bekanntmachung des Hinweises auf die Patenterteilung:
**13.04.83 Patentblatt 83/15**

(84) Benannte Vertragsstaaten:
**BE CH FR GB IT NL SE**

(56) Entgegenhaltungen:
**DE-A-2 023 716**
**DE-A-2 255 578**
**FR-A-1 541 435**
**FR-A-2 212 667**
**US-A-3 667 057**
**US-A-3 846 692**
**US-A-3 989 051**

(73) Patentinhaber: **SIEMENS AKTIENGESELLSCHAFT,**
**Berlin und München Wittelsbacherplatz 2,**
**D-8000 München 2 (DE)**

(72) Erfinder: **Kusserow, Bernd, Kulmbacher Strasse 12,**
**D-8520 Erlangen (DE)**

Verfahren und Vorrichtung zur Messung von elektrischen Strömen oder Spannungen in einer Mehrzahl von Strom- bzw. Spannungskreisen

Strom- oder Spannungsmessungen sind relativ unproblematisch, solange die Spitzenwerte nicht allzu langsamen Amplitudenschwankungen unterworfen sind. Treten jedoch solche langsamen Amplitudenschwankungen auf, so müssen Spitzenwertmesser mit entsprechend großer Zeitkonstante eingesetzt werden, damit die Amplitudenschwankungen die Spitzenwertmessung nicht beeinträchtigen. Die geforderte Zeitkonstante kann dabei so groß sein, daß Änderungen im Signalverhalten, die insbesondere aus technischen Fehlern vom Spitzenwert nicht erfaßt werden. Dies hätte zur Folge, daß weiter ein großer Spitzenwert angezeigt wird, obwohl der Strom mittlerweile auf einen Bruchteil dieses Wertes oder ganz auf Null abgesunken ist.

In der allgemeinen Technik ist es für verschiedene Anwendungsfälle notwendig, Strom- oder Spannungskreise zu überwachen. Aus der FR-A-1 541 435 ist ein Verfahren zum Überwachen von elektrischen Spannungen in einer Mehrzahl von Spannungskreisen bekannt, wobei die Spannung eines jeden Kreises mit einem Vergleichswert verglichen wird und wobei Ausgangssignale bei Nichtgleichheit der Spannungswerte mit einem Vergleichswert an den Spannungskreisen zugeordneten Anzeigemitteln zur Anzeige gebracht werden, bei dem jeder separat erfaßte Spannungswert an einem ihm eigens zugeordneten Komparator speziell mit einem solchen Vergleichswert verglichen wird, der als Mittelwert aus den Spannungswerten sämtlicher beteiligter Spannungskreise gebildet ist. Dieses Verfahren wird speziell zur Meldung und Beseitigung von Kurzschlüssen hintereinandergeschalteter Elektrolysezellen verwendet. Als Komparatoren dienen dabei üblicherweise Schaltstufen.

Ein Gebiet, wo obige Probleme aber besonders relevant sind, ist die elektromedizinische Interferenzstromtherapie. Hier, wo immer mit mehreren Strom- bzw. Spannungskreisen gearbeitet wird (z. B. die stereodynamische Methode nach US-A-4 023 574), ist speziell die Anzeige des jeweiligen Spitzenwertes eines jeden Kreises notwendig. Für die verwendeten Spitzenwertmesser muß dann jedoch die Zeitkonstante sehr groß sein (wenigstens im Minutenbereich), damit bei Parallelschaltung der Kreise die auftretenden Amplitudenschwankungen sehr niedriger Frequenz (ca. 0,01 /s) nicht das Anzeigeergebnis verfälschen. Bei einem Spitzenwertmesser mit solch großer Zeitkonstante treten jedoch dann mit Sicherheit immer die beschriebenen Nachteile auf. Eine Änderung des Übergangswiderstandes Elektrode zur Haut, beispielsweise durch Lösen einer Elektrode, oder das völlige Abfallen der Elektrode kann also nicht angezeigt werden. Auch eine Änderung der Intensität mittels Intensitätsregler könnte nicht kontrolliert werden.

Speziell aus der DE-A-2 255 578 ist es nun bei einem Apparat zur therapeutischen Behandlung mit Summenströmen bekannt, dem Ausgang eines Differenzverstärkers ein Anzeigemittel derart nachzuschalten, daß es erst bei Überschreiten einer vorgebbaren Grenzspannung des Verstärkers aktiviert wird. Das Anzeigemittel ist durch eine einer Zenerdiode nachgeschalteten Lumineszenzdiode realisiert.

Aufgabe vorliegender Erfindung ist es nun, eine Möglichkeit zu schaffen, mit der insbesondere rasche Änderungen im Signalverhalten erfaßt und angezeigt werden können.

Die Aufgabe wird durch die Gesamtheit der im Patentanspruch 1 angegebenen Merkmale gelöst. In Weiterbildung dieser Merkmale entsprechend dem Patentanspruch 4 kann beim erfindungsgemäßen Verfahren eine rasche Korrektur der Spitzenwertanzeige erfolgen.

Bei einer Vorrichtung zur Durchführung des Verfahrens sind als Komparatoren Operationsverstärker mit den Ausgängen der Strom- bzw. Spannungskreise zusammengeschaltet in dem Sinne, daß jeder Kreisausgang einerseits separat mit dem einen Eingang und andererseits über einen gemeinsamen Mittelwertbildner auch mit dem anderen Eingang des zugehörigen Operationsverstärkers verbunden ist. Dagegen sind die Komparatoren beim Stand der Technik mono- oder bistabile Schaltstufen.

Sind die mit dem erfindungsgemäßen Verfahren gemessenen Ströme bzw. Spannungen allesamt gleich groß oder weichen sie lediglich unwesentlich in vorgebbarer Grenze voneinander ab, so befinden sich sämtliche Komparatoren im gleichen Schaltzustand. Weichen hingegen Strom bzw. Spannung wenigstens einer der Kreise von Strom- bzw. Spannungswerten der anderen Kreise ab, und überschreitet die Abweichung die vorgegebene Grenze, so schaltet der entsprechende Komparator in den anderen Schaltzustand um. Das abweichende Verhalten des Strom- bzw. Spannungskreises wird durch Ansprechen des zugehörigen Indikators angezeigt.

Es empfiehlt sich den Mittelwert aus den Strom- bzw. Spannungswerten sämtlicher beteiligter Strom- bzw. Spannungskreise um etwa 10 bis 20% zu reduzieren. Am Komparator erfolgt dieses am zweckmäßigsten dadurch, daß am Mittelwerteingang der Mittelwert durch Spannungsteilung um diesen erwünschten Prozentsatz gegenüber dem Istwert abgesenkt wird.

Sind die mit einem solchen Verfahren gemessenen Ströme bzw. Spannungen sämtlich gleich groß oder weichen sie lediglich unwesentlich in vorgebbarer Grenze voneinander ab, so befinden sich sämtliche Komparatoren im gleichen Schaltzustand. Weichen hingegen Strom bzw. Spannung wenigstens einer der Kreise von Strom- bzw. Spannungswerten der anderen Kreise ab, und überschreitet die Abweichung die vorgegebene Grenze, so schaltet der entsprechende Komparator in den anderen Schaltzustand um. Das abweichende Verhalten des Strom- bzw. Spannungskreises wird durch Ansprechen des zugehörigen Indikators angezeigt. Die Anzeige erfolgt also mindestens immer dann, wenn der gemessene Strom- bzw. Spannungswert den Mittelwert der Ströme bzw. Spannungen sämtlicher Kreise um einen solchen Prozentsatz unterschreitet, der bei etwa 10 bis 20% liegt. Dies erfolgt zweckmäßigerweise dadurch, daß am Komparator der Mittelwert durch Spannungsteilung um den erwünschten Prozentsatz gegenüber dem Istwert abgesenkt wird.

Mit der Anzeige der geänderten Strom- bzw. Spannungsverhältnisse an einem Strom- bzw. Spannungskreis kann erfindungsgemäß auch eine Korrektur der Spitzenwertanzeige vorgenommen werden. Dazu wird der als Vergleichswert gebildete Mittelwert sämtlicher beteiligter Strom- bzw. Spannungskreise auf einen Spitzenwertmesser mit Speicherglied gegeben, wobei gleichzeitig sämtliche Ausgangssignale der Komparatoren mit einer Entladeschaltung für das Speicherglied zu dessen Entladung bei geändertem Mittelwert gegeben werden. Darüber hinaus ist es vorteilhaft, eine weitere Entladeschaltung zur Entladung des Speichergliedes in Abhängigkeit von Intensitätsänderungen an einem Intensitätsregler vorzusehen. In diesem Falle wird also bei jeder gewollten Intensitätsänderung der Spitzenwert eines Spitzenwertmessers mit an sich sehr großer Zeitkonstante rasch an den tatsächlichen Istwert angepaßt.

Weitere Vorteile und Einzelheiten der Erfindung ergeben sich aus der nachfolgenden Beschreibung eines Ausführungsbeispiels der Erfindung in Verbindung mit den weiteren Unteransprüchen.

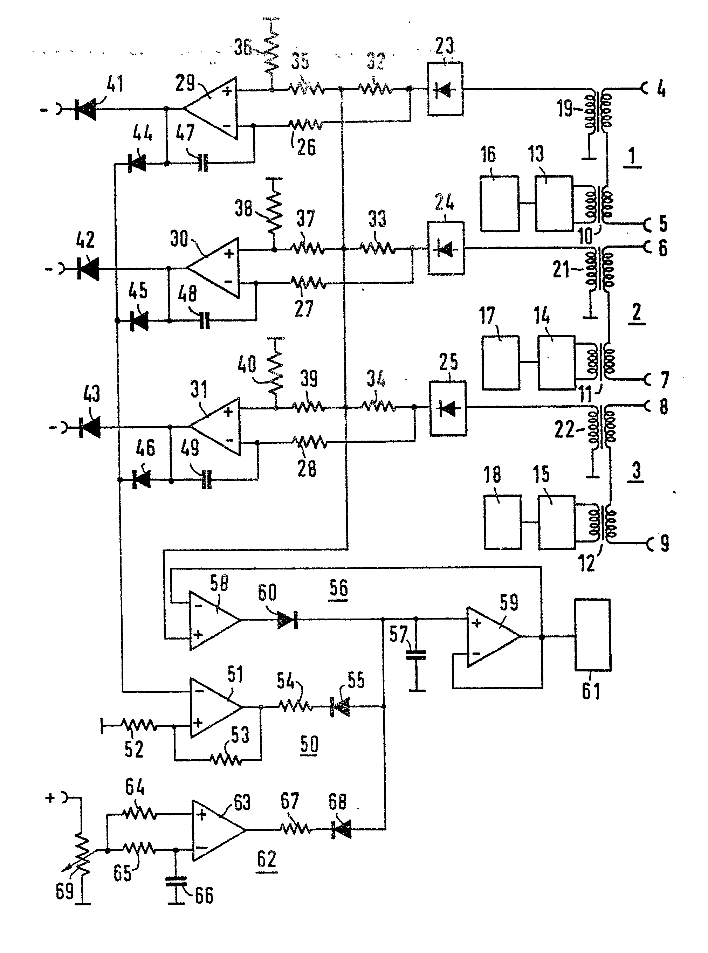

In der Figur, die ein Ausführungsbeispiel im Prinzipschaltbild zeigt, sind mit 1, 2 und 3 Stromkreise für die Interferenzstromtherapie bezeichnet. Die Klemmen 4 bis 9 deuten die Anschlüsse für die Paare an benötigten Reizstromelektroden an. Die Speisung der einzelnen Kreise 1, 2 bzw. 3 erfolgt durch transformatorische Ankopplungen 10, 11 bzw. 12 über Gegentaktstufen 13, 14, 15 in Abhängigkeit von Mittelfrequenzgebern 16, 17, 18. Die einzuspeisenden Ströme liegen im Mittelfrequenzbereich zwischen 1000 und 100.000 Hz.

Die Abnahme der Strom- bzw. Spannungswerte an den einzelnen Stromkreisen 1, 2, 3 erfolgt mittels weiterer induktiver Ankopplung 19, 21 bzw. 22. Die so abgenommenen Strom- bzw. Spannungswerte werden in Gleichrichterschaltungen 23, 24, 25 gleichgerichtet. Nach Gleichrichtung erfolgt die Zuleitung zu Operationsverstärkern 29, 30, 31, die als Komparatoren zum Vergleich mit einem Mittelwert dienen. Die gleichgerichteten Strom- bzw. Spannungswerte werden dabei den invertierenden Eingängen eines jeden Operationsverstärkers 29, 30 bzw. 31 über einen Widerstand 26, 27 bzw. 28 direkt zugeleitet. Die Signale werden jedoch gleichzeitig auch durch Parallelschaltung über die Widerstände 32, 33 und 34 zum Mittelwert geführt. Das Mittelwertsignal wird dann über Spannungsteiler 35, 36 bzw. 37, 38 bzw. 39, 40 auf die nicht invertierenden Eingänge der Operationsverstärker 29, 30 bzw. 31 gegeben. Die Dimensionierung der einzelnen Spannungsteiler ist so gewählt, daß durch sie der jeweils anstehende Mittelwert der Strom- bzw. Spannungswerte sämtlicher beteiligter Kreise 1, 2, 3 am nichtinvertierenden Eingang eines jeden Operationsverstärkers 29 bis 31 um ca. 10 bis 20% gegenüber dem Istwert abgesenkt ist. Im Vergleich Signalwert des Stromes bzw. der Spannung mit dem Mittelwert ergibt sich also ein Ausgangssignal am Ausgang eines Operationsverstärkers 29, 30 oder 31 dann, wenn der Strom bzw. Spannungswert eines der Kreise den gemessenen Mittelwert sämtlicher beteiligter Kreise um die gewählte Grenze zwischen 10 bis 20% unterschreitet. Das damit am Ausgang des Operationsverstärkers 29, 30 oder 31 auftretende Signal wird mittels Leuchtdiode 41, 42 bzw. 43 am Ausgang der Operationsverstärker zur Anzeige gebracht.

Gleichzeitig wird auch über eine ausgangsseitig über Dioden 44 bis 46 angeschaltete Entladevorrichtung der Spitzenstromwert eines Spitzenwertmesser nach unten korrigiert. Ein Flackern der Leuchtanzeige der Leuchtdioden 41, 42 oder 43 bei kurzzeitigem Stromrückgang wird durch Integrationskondensatoren 47 bzw. 49 verhindert. Die Entladevorrichtung ist mit 50 bezeichnet.

Sie umfaßt einen Operationsverstärker 51 mit den Beschaltungswiderständen 52, 53, 54 und Entladediode 55. Der Spitzenwertmesser 56 umfaßt den zu entladenden Speicherkondensator 57. Dieser ist eingangsseitig über einen Operationsverstärker 58 mit Diode 60 am Mittelwerteingang der Operationsverstärker 29, 30 und 31 angeschaltet. Ankoppelglied zur Ankopplung an die Entladeschaltung 50 ist die Diode 55. Die Anzeige des jeweiligen Spitzenwertes erfolgt über den Operationsverstärker 59 am Anzeige- bzw. Registriergerät 61.

Eine Entladung des Ladespeichers 57 im Spitzenwertmesser 56 erfolgt auch dann, wenn die Intensität der einzuspeisenden Ströme gewollt geändert wird. Eine geeignete Entladeschaltung 62 umfaßt wieder einen Operationsverstärker 63 mit Beschaltungswiderständen 64, 65, 67 und Entladediode 68. Der invertierende Eingang des Operationsverstärkers 63 liegt ferner an einer Verzögerungskapazität 66. Beide Eingänge sind gemeinsam am Intensitätsregler 69 angeschaltet. Wird also der Patientenstrom durch Zurückdrehen des Intensitätsreglers verkleinert, so wird auch die Spitzenwertanzeige automatisch an den verkleinerten Wert angepaßt. Die Verzögerungskapazität 66 bewirkt dabei im Zusammenspiel mit dem Widerstand 65, daß Intensitätsvergrößerungen durch Hochdrehen des Reglers 69 das Signalverhalten des Operationsverstärkers 63 am Ausgang nicht beeinflussen; der Ausgang des Operationsverstärkers bleibt auf positivem Potential. Erst ein Herunterregeln der Intensität bewirkt Umschaltung auf negatives Potential während der Regelzeitdauer und damit Spitzenwertentladung im erwünschten Sinne.

## Patentansprüche

1. Verfahren zum Überwachen von elektrischen Strömen oder Spannungen in einer Mehrzahl von Strom- bzw. Spannungskreisen, insbesondere der Mittelfrequenzstromkreise bei der elektromedizinischen Interferenzstromtherapie, wobei Strom oder Spannung eines jeden Kreises (1, 2, 3) mit einem Vergleichswert verglichen werden und wobei Ausgangssignale bei Nichtgleichheit der Strom- bzw. Spannungswerte mit dem Vergleichswert an den Strom- bzw. Spannungskreisen (1, 2, 3) zugeordneten Anzeigemitteln (41, 42, 43) zur Anzeige gebracht werden und bei dem jeder separat erfaßte Strom- oder Spannungswert an einem ihm eigens zugeordneten Komparator (29, 30, 31) speziell mit einem solchen Vergleichswert verglichen wird, der als Mittelwert aus den Strom- oder Spannungswerten sämtlicher beteiligter Strom- bzw. Spannungskreise gebildet und um einen vorgebbaren Betrag reduziert wird, so daß bei Überschreiten dieser speziell vorgegebenen Grenze das dem betreffenden Strom- bzw. Spannungskreis (1, 2, 3) zugeordnete Anzeigemittel (41, 42, 43) aktiviert wird.

2. Verfahren nach Anspruch 1, dadurch gekennzeichnet, daß die Strom- bzw. Spannungswerte jedes Kreises (1, 2, 3) dem einen Eingang eines diesem Kreis als Komparator zugeordneten Operationsverstärkers (29, 30, 31) zugeleitet werden, während der andere Eingang des jeweiligen Operationsverstärkers (29, 30, 31) mit dem Vergleichswert beaufschlagt wird.

3. Verfahren nach Anspruch 1 oder 2, dadurch gekennzeichnet, daß die Reduktion des Mittelwertes durch Spannungsteilung erfolgt in dem Sinne, daß der Mittelwert um einen vorgebbaren Prozentsatz gegenüber dem Istwert, vorzugsweise zwischen 10 und 20%, abgesenkt wird.

4. Verfahren nach einem der Ansprüche 1 bis 3, dadurch gekennzeichnet, daß der als Vergleichswert gebildete Mittelwert aus den Strom- oder Spannungswert sämtlicher beteiligter Strom- bzw. Spannungskreise (1, 2, 3) auf einen Spitzenwertmesser (56) mit Speicherglied (57) gegeben wird, wobei gleichzeitig sämtliche Ausgangssignale der Komparatoren (29, 30, 31) auf eine Entladeschaltung (50) für das Speicherglied (57) zu dessen Entladung bei geändertem Mittelwert gegeben werden.

5. Vorrichtung zur Durchführung des Verfahrens nach einem der Ansprüche 1 bis 4, dadurch gekennzeichnet, daß als Komparatoren Operationsverstärker (29, 30, 31) mit den Ausgängen der Strom- bzw. Spannungskreise (1, 2, 3) zusammengeschaltet sind in dem Sinne, daß jeder Kreisausgang einerseits separat mit dem einen Eingang und andererseits über einen gemeinsamen Mittelwertbildner (32, 33, 34) auch mit dem anderen Eingang des zugehörigen Operationsverstärkers verbunden ist.

6. Vorrichtung nach Anspruch 5, dadurch gekennzeichnet, daß zur Mittelwertbildung die Ausgänge sämtlicher beteiligter Kreise (1 bis 3) über Widerstände (32 bis 34) parallelgeschaltet sind.

7. Vorrichtung nach Anspruch 5 oder 6, dadurch gekennzeichnet, daß eine weitere Entladeschaltung (62) zur Entladung des Speichergliedes (57) des Spitzenwertmessers (56) in Abhängigkeit von Intensitätsänderungen an einem Intensitätsregler (69) vorhanden ist.

8. Vorrichtung nach einem der Ansprüche 5—7, dadurch gekennzeichnet, daß die weitere Entladeschaltung (62) einen Operationsverstärker (63) umfaßt, der ausgangsseitig über eine Entladediode (68) mit dem Speicherglied (57) des Spitzenwertmessers (56) und eingangsseitig über wenigstens ein RC-Glied (65, 66) am der Polaritätsrichtung der Entladediode (68) gemäßen Eingang mit dem Intensitätsregler (69) verbunden ist.

## Claims

1. A method of monitoring electric currents or voltages in a plurality of current or voltage circuits, as the case may be, in particular medium frequency electromedical interference current therapy, wherein the current or voltage of each circuit (1, 2, 3) is compared with a comparison value and any non-conformity of the current or voltage values with the comparison value causes output signals to be displayed at display means assigned to current or voltage circuits (1, 2, 3) and wherein each separately determined current or voltage value is specifically compared with such a comparison value in a comparator (29, 30, 31) particularly assigned to the current or voltage value, which comparison value is formed as an average value from the current or voltage values of all participating current or voltage circuits and reduced by a predeterminable amount, so that when this specifically predetermined limit is exceeded, the display means (41, 42, 43) assigned to the respective current or voltage circuit (1, 2, 3) is activated.

2. A method as claimed in Claim 1, characterised in that the current or voltage values of each circuit (1, 2, 3) are fed to one input of an operational amplifier (29, 30, 31) assigned to this circuit as a comparator, whereas the other input of the respective operational amplifier (29, 30, 31) is supplied with the comparison value.

3. A method as claimed in Claim 1 or 2, characterised in that the reduction of the mean value is carried out by voltage division, the mean value being reduced by a predeterminable percentage in relation to the theoretical value, preferably between 10 and 20%.

4. A method as claimed in one of Claims 1 to 3, characterised in that the mean value formed as a comparison value from the current or voltage values of all participating current or voltage circuits (1, 2, 3) is fed to a peak value measuring device (56) having a storage element (57), where all output signals of the comparators (29, 30, 31) are simultaneously fed to a discharge circuit (50) for the storage element (57) to discharge it in the event of a changed mean value.

5. An arrangement for carrying out the method as claimed in one of Claims 1 to 4, characterised in that operational amplifiers (29, 30, 31) are interconnectef with the outputs of the current or voltage circuits (1, 2, 3) as comparators in such manner that, on the one hand, each circuit output is separately connected to the one input and, on the other hand, it is also connected via a common mean value forming stage (32, 33, 34) to the other input of the assigned operational amplifier.

6. An arrangement as claimed in Claim 5, characterised in that for mean value formation, the outputs of all participating circuits (1 to 3) are connected in parallel via resistors (32 to 34).

7. An arrangement as claimed in Claim 5 or 6, characterised in that there is provided a further discharge circuit (62) for the discarge of the storage element (57) of the peak value measuring device (56) in dependence upon changes in intensity in an intensity regulator (69).

8. An arrangement as claimed in one of Claims 5 to 7, characterised in that the further discharge circuit (62) comprises an operational amplifier (63) which is connected at the output end via a discharge diode (68) to the storage element (57) of the peak value measuring device (56), and at the input end, is connected to the intensity regulator (69) via at least one RC-element (65, 66) at the input of the discharge diode (69) in accordance with the direction of polarity thereof.

**Revendications**

1. Procédé pour surveiller des courants ou tensions électriques dans une pluralité de circuits de courant ou de tension, notamment le circuit de courant de fréquences moyennes dans le cas de la thérapie électromédicale par courants d'interférences, le courant ou la tension de chaque circuit (1, 2, 3) étant comparé à une valeur de comparaison, et dans le cas d'une non égalité des valeurs de courant de tension avec la valeur de comparaison, des signaux de sortie étant affichés sur des moyens d'affichage (41, 42, 43) associés aux circuits de courant ou de tension (1, 2, 3), et dans lequel chaque valeur de courant ou de tension détectée séparément est comparée en particulier, dans un comparateur (29, 30, 31) qui lui est associé de façon propre, avec une valeur de comparaison qui est formée en tant que valeur moyenne à partir des valeurs de courant ou de tension de tous les circuits de courant ou de tension intéressés, et qui est réduite d'une valeur pouvant être prédéterminée, de manière que lors du dépassement de cette limite prédéterminée de façon particulière, le moyen d'affichage (41, 42, 43) associé au circuit de courant ou de tension (1, 2, 3) concerné, soit activé.

2. Procédé suivant la revendication 1, caractérisé par le fait que les valeurs de courant ou de tension de chaque circuit (1, 2, 3) sont appliquées à une des entrées d'un amplificateur opérationnel (29, 30, 31) associée à ce circuit en tant que comparateur, tandis que l'autre entrée de l'amplificateur opérationnel respectif (29, 30, 31) reçoit la valeur de comparaison.

3. Procédé suivant l'une des revendications 1 ou 2, caractérisé en ce que la réduction de la valeur moyenne par division de tension a lieu dans le sens que la valeur moyenne est réduite d'un pourcentage pouvant être prédéterminé par rapport à la valeur réelle, de préférence entre 10 et 20%.

4. Procédé suivant l'une des revendications 1 à 3, caractérisé en ce que la valeur moyenne formée en tant que valeur de comparaison à partir des valeurs de courant ou de tension de tous les circuits de courant ou de tension intéressés (1, 2, 3) est appliquée à un dispositif (56) de mesure de valeur de crête comportant un élément de mémorisation (57), tous les signaux de sortie des comparateurs (29, 30, 31) étant appliqués simultanément au circuit de décharge (50) pour l'élément de mémorisation (57), pour le décharger lors d'une valeur moyenne modifiée.

5. Dispositif pour la mise en oeuvre du procédé suivant l'une des revendications 1 à 4, caractérisé en ce que, en tant que comparateurs, des amplificateurs opérationnels (29, 30, 31) sont reliés aux sorties des circuits de courant ou de tension (1, 2, 3), dans le sens que chaque sortie de circuit est reliée d'une part séparément à une des entrées et d'autre part, par l'intermédiaire d'un circuit de formation de valeur moyennes (32, 33, 34) commun, également à l'autre entrée de l'amplificateur opérationnel associée.

6. Dispositif suivant la revendication 5, caractérisé en ce que pour la formation de la valeur moyenne les sorties de tous les circuits intéressés (1 à 3) sont reliés en parallèle par l'intermédiaire de résistances (32 à 34).

7. Dispositifs suivant l'une des revendications 5 ou 6, caractérisé par le fait qu'il existe un autre circuit de décharge (62), pour décharger l'élément de mémorisation (57) du dispositif (56) de mesure de la valeur de crête, en fonction des variations d'intensité au niveau d'un régulateur d'intensité (69).

8. Dispositif suivant l'une des revendications 5 à 7, caractérisé par le fait que l'autre dispositif de décharge (62) comporte un amplificateur opérationnel (63) qui est relié côté sortie à l'élément de mémorisation (57) du dispositif (56) de mesure de valeurs de crête, par l'intermédiaire d'une diode de décharge (68), et côté entrée à l'entrée, conforme à la polarité de la diode de décharge (68), du régulateur d'intensité (69), par l'intermédiaire d'au moins un élément RC (65, 66).